# EUROPEAN PATENT SPECIFICATION

(11) **EP 2 222 316 B1**
(45) Date of publication and mention of the grant of the patent: **14.05.2014**
(21) Application number: 08868161.4
(22) Date of filing: 28.11.2008
(51) Int. Cl.: A61K 35/60, A61P 17/00, A61P 27/00

(54) **USE OF PARTLY HYDROLYZED FISH GELATIN FOR THE PREPARATION OF TOPICALLY ADMINISTERED PHARMACEUTICAL COMPOSITIONS**
VERWENDUNG VON TEILWEISE HYDROLYSIERTER FISCHGELATINE ZUR HERSTELLUNG VON TOPISCH ANGEWENDETEN PHARMAZEUTISCHEN ZUSAMMENSETZUNGEN
UTILISATION DE GÉLATINE DE POISSON PARTIELLEMENT HYDROLYSÉE POUR LA PRÉPARATION DE COMPOSITIONS PHARMACEUTIQUES ADMINISTRÉES DE MANIÈRE TOPIQUE

(30) Priority: 28.12.2007 IT RM20070680
(43) Date of publication of application: 01.09.2010
(73) Proprietor: Silvestrini, Bruno, 00179 Roma (IT); Bonanomi, Michele, 00198 Roma (IT)
(72) Inventor: Silvestrini, Bruno, 00179 Roma (IT); Bonanomi, Michele, 00198 Roma (IT)
(74) Representative: Biggi, Cristina
(86) International application number: PCT/IB2008/003266
(87) International publication number: WO 2009/083768

(56) References cited:
- EP-A1- 1 273 239
- WO-A1-2004/075871

## Description

The object of the present invention is the use of partly hydrolyzed fish gelatin for the preparation of pharmaceutical compositions for topical use, intended for the treatment of pathologies of the oropharyngeal and gingival, ocular, dermatological, gynaecological, proctological, otologic and rhinological regions.

It is known that the fish gelatin is an amino acid source, which are essential growth factors for the cells. *In vitro* tests on cell cultures have shown a good trophic action of the fish gelatin added to the culture broth.

Compared with an equipollent amino acid mixture, the fish gelatin has the advantage of providing its own amino acids stepwise, by improving in this way the trophic effects and extending the action duration thereof. Properties of the fish gelatin, extracted from the skin of deep water sea fishes, such as for example cods, are not comparable or transferable to the called "animal" or "mammalian" gelatin, extracted from bones and skin of cattle and swines. The fish skin, in fact, has significant structural and biochemical differences relative to that of a mammal as a consequence of the different phylogenetic path undertook by the two classes of living creatures.

A not negligible advantage provided by the fish gelatin is to be connected to the regulatory restrictions adopted towards the animal gelatin, which is considered at risk because of the spongiform encephalopathy (BSE) and, therefore, it is to be avoided, where possible, above all in food and pharmaceutical fields. The fish gelatin can be used as such or in a completely of partially hydrolyzed form.

The completely hydrolyzed gelatin is obtained by a forced hydrolytic treatment in the presence of an excess of mineral acids and relatively high temperatures. Normally, the complete hydrolysis (which leads to amino acids) is carried out in the presence of concentrated hydrochloric acid, in an autoclave, at 110°-120°C, for a time between 12 and 20 hours.

On the contrary, the partly hydrolyzed gelatin, object of the present invention, is obtained by subjecting the crude fish gelatin to a controlled acidic treatment: the same is commonly defined "type A" gelatin. Said treatment modifies the native original structure and leads to the hydrolysis of inter-chain linkages between the protein chains of the collagen, to give an end product in form of straight chains or fibrils, having an average molecular weight of 5,000 D. Furthermore, said gelatin has a protein content equal to 97% (Rousselot specifications) and has no free amino acids.

Said treatment includes:
1) repeatedly extracting the crude fish gelatin with water, in an acid environment (at a pH preferably between 4 and 6), at a progressively increasing temperature (from 50°C to about 80°C);
2) after an optional clarification or decolorization of the above aqueous extract, filtering and evaporating the water under controlled conditions, preferably by spray drying.

Aspect, color, smell, pH, isoelectric point, viscosity, loss during the drying, impurities must be in conformity with the Pharmacopoeia specifications (such as, for example, those reported in Eur. Ph., V edition, 2005, page 1651).

The partly hydrolyzed fish gelatin offers various advantages with respect to both the animal and the non-hydrolyzed fish gelatins. In particular, the partly hydrolyzed fish gelatin is characterized by a greater handleability and it is preferred for a better water solubility and for the absence of gelation phenomena

(typical of the non-hydrolyzed mammalian gelatin and fish gelatin); consequently, said gelatin allows stable solutions. Furthermore, the partially hydrolyzed fish gelatin, topically administered, exerts a trophic action definitely higher than that of the non-hydrolyzed gelatin.

Previous proposals for the use of the partly hydrolyzed fish gelatin concern both systemic treatments (EP 1407677) and potential topical uses of compositions (mixtures with polysaccharides) capable of manifesting gelling properties (WO 2006/123173).

To date, the gelatin is commonly used as an additive, carrier, excipient, coating agent, binder, gelling agent.

On the contrary, it is not known the administration as a medicament, i.e. as an active ingredient, of the partly hydrolyzed fish gelatin, as such or in combination with other active ingredients having a pharmacological activity, in topical preparations, for the purpose of therapeutically using its trophic properties. In particular, the partly hydrolyzed fish gelatin is better both than the non-hydrolyzed gelatin, and equivalent mixtures of free amino acids: with respect to the first one, it involves a greater release of the amino acids, while with respect to the second ones, it allows a gradual and sustained release of the amino acids, with apparent advantages above all with reference to the trophic action.

In other words, a trophic action of the partly hydrolyzed fish gelatin in topical applications (wherein said therapeutic activity is directly and exclusively exerted on the tissue treated for this administration route) is not known.

There is a great need, in medicine, of being able to exert a specific trophic-therapeutic action for a topical administration, by reducing in this way both the quantity of active ingredient required for the obtainment of the desired effect, and the side or toxic effects, if any, imputable to the systemic administrations.

The present invention provides an answer to the need above mentioned, as it is apparent from the following detailed description.

Applicants have unexpectedly found that a composition for topical administration, including a proper quantity of partly hydrolyzed fish gelatin, is capable of giving an adequate answer to the problems above pointed out.

It is an object of the present invention the use of the partly hydrolyzed fish gelatin as an active ingredient for the preparation of topical pharmaceutical compositions (namely, to be locally administered), as reported in the appended independent claim.

Preferred embodiments of the present invention are reported in the appended dependent claims.

The present invention relates to the use of the partly hydrolyzed fish gelatin in pharmaceutical preparations intended for the topical treatment of some pathological conditions. Preferably, said conditions belong to one or more of the following disease fields: oropharyngeal, ocular, dermatological, gynaecological, proctological, otologic, rhinological fields.

In said pharmaceutical preparations, the partly hydrolyzed fish gelatin has completely unexpectedly shown to possess a marked and specific local trophic (i.e., topical) action towards the tissue on which it has been applied.

In said topical pharmaceutical preparation, the partly hydrolyzed fish gelatin exists, as an active ingredient, in an effective quantity for treating the pathology of interest.

In a preferred embodiment of the invention, the partly hydrolyzed fish gelatin exists in a quantity between 0.05% and 10% by weight, based on the total weight of the composition; more preferably, between 0.1% and 5% by weight.

In another preferred embodiment of the invention, said pharmaceutical composition also includes at least another pharmaceutical active ingredient selected from the group including: antibiotics, cortisone drugs, antihistamines, vasoconstrictors, vitamins, with a particular respect to the retinoides, and others, for the purpose of improving the activity, the stability and the tolerability and to extend the permanence in the application site.

In another preferred embodiment of the invention, said pharmaceutical composition further includes at least a pharmaceutically acceptable additive, excipient, carrier, preservative.

By way of example, in a first preferred embodiment of the invention, the partly hydrolyzed fish gelatin is used as an active ingredient for the preparation of a stomatological or ophtalmological pharmaceutical composition, for topically treating pathological conditions characterized by dryness of the oropharyngeal mucosa and the ocular mucosa (for example: dry mouth, dry eye, allergic conjunctivitis). Said pharmaceutical compositions are in a suitable form for administration in stomatology or ophtalmology.

In said embodiment, the partly hydrolyzed fish gelatin is preferably used for the preparation of a solution to be used as a salivary or lacrimal substitute for topically treating pathological states characterized by dryness of the oropharyngeal or ocular mucosa (simple salivary or lacrimal substitutes).

In a preferred embodiment, the above solutions further include at least a thickening/viscosity agent; for example, hydroxypropyl cellulose, hydroxypropylmethyl cellulose, polyvinyl alcohol.

In another preferred embodiment, said solutions further include at least an isotonic mineral salt, for example sodium chloride.

In another preferred embodiment, said solutions further include one or more pH-stabilizing substances; said substances are selected, for example, from the group including: sodium citrate, sodium borate, monobasic potassium phosphate/bibasic sodium phosphate.

In another preferred embodiment of the invention, the partly hydrolyzed fish gelatin is used as an active ingredient for the preparation of a gynaecological pharmaceutical composition, for topically treating pathological states in gynecology (for example: vaginites).

Said pharmaceutical composition is in a form suitable for administration in gynecology (for example: vaginal douche, pessary, cream).

In said embodiment, the partly hydrolyzed fish gelatin is preferably used for the preparation of an aqueous solution to be used as a vaginal douche.

In a preferred embodiment, said douche further includes at least a substance having an antibacterial and/or preservative activity, such as, for example: sorbic acid, benzoic acid, benzalkonium chloride, parabens.

In another preferred embodiment, said douche further includes an opportune quantity of lactic acid.

In another particularly preferred embodiment for gynaecological use, the partly hydrolyzed fish gelatin is used, as active ingredient, for the preparation of a pessary, wherein said pessary includes a solution of said fish gelatin in semi-synthetic triglycerides.

In a further preferred embodiment of the invention, the partly hydrolyzed fish gelatin is used as an active ingredient for the preparation of a dermatological pharmaceutical composition, for topically treating pathological states in dermatology (for example: dystrophic ulcers, dermatites, atopical eczemas, sunburns).

Said pharmaceutical composition is in a suitable form for skin administration (for example: dermatological cream, cicatrizant cream).

In a preferred embodiment, said cream further includes at least a vitamin (selected, for example, from: vitamin A and/or E and/or C) and a cicatrizant agent, such as hyaluronic acid.

In another preferred embodiment of the invention, the partly hydrolyzed fish gelatin is used as an active ingredient for the preparation of a proctological pharmaceutical composition, for topically treating pathological states in proctology (for example: haemorrhoidal states, rhagades, proctites).

Said pharmaceutical composition is in a suitable form for proctological administration (for example: cream or ointment). In this case, preferably, said formulation is packed in a proper tube with a dispenser.

By way of exemplifying and example of the invention, there are hereinafter reported some examples of preferred topical pharmaceutical compositions of the invention including, as an active ingredient, partly hydrolyzed fish gelatin according to what previously described.

Such preferred topical pharmaceutical compositions of the invention are prepared with methodologies traditionally employed in the pharmaceutical technology; however, for each of them, a short description is given.

### Example 1 - Salivary or lacrimal substitute

| | |
|---|---|
| Hydroxypropylmethyl cellulose (or analogous viscosity means) | 0.700 g |
| Partly hydrolyzed fish gelatin | 0.100 g |
| Sodium chloride | 0.450 g |
| Monobasic sodium phosphate | 0.240 g |
| Bibasic sodium phosphate | 0.663 g |
| Benzalkonium chloride (preservative) | 0.015 g |
| Sterile distilled water | q.s. to 100 ml |

Hydroxypropylmethyl cellulose is swollen in sterile distilled water, fish gelatin is added and successively the mixture of pH-stabilizing salts, sodium chloride and finally the preservative is added.

### Example 2 Douche

| | |
|---|---|
| Partly hydrolyzed fish gelatin | 0.150 g |
| Bisabolol | 0.100 g |
| Chitoglycan (water-soluble chitosan) | 1.5 g |
| Liquid germall plus® (preservatives mixture) | 0.100 g |
| Lactic acid | q.s. to pH 4.5 |
| Water | q.b. to 100 ml |

Chitosan is dissolved in water, fish gelatin and bisabolol are added, pH is corrected to 4.5 with lactic acid and finally the preservative is added.

### Example 3 Pessary

| | |
|---|---|
| Partly hydrolyzed fish gelatin | 0.200 g |
| Semisynthetic triglycerides | 1.900 g |

It is prepared with a traditional methodology, by adding the fish gelatin to the melted triglycerides, operating at 35-40°C.

### Example 4 Cicatrizant dermatological cream

| | |
|---|---|
| Partly hydrolyzed fish gelatin | 1.0 g |
| Filamentous Vaseline | 5.0 g |
| White mineral oil | 10 g |
| Cetylstearyl alcohol | 9.0 g |
| Olive oil | 4.0 g |
| Vitamin E | 0.2 g |
| Vitamin A | 0.1 g |
| Germaben II^{®} (preservative) | 0.2 g |
| Water | q.s. to 100 g |

The cetylstearyl alcohol and filamentous vaseline mixture is melted and the white mineral oil, the olive oil (containing the two vitamins) and finally the fish gelatin dissolved in water and the preservative are added.

### Example 5 Proctological cream (ointment)

| | |
|---|---|
| Partly hydrolyzed fish gelatin | 2.5 g |
| Filamentous vaseline | 5.0 g |
| White mineral oil | 10 g |
| Lanolin | 12 g |
| Polysorbate 80 | 10 g |
| Glycerine | 12 g |
| Germall plus^{®} (preservative) | 0.2 g |
| Water | q.s. to 100 g |

Polysorbate is added to the fat base (white mineral oil, filamentous vaseline and lanolin) and, under stirring, the partly hydrolyzed gelatin (in a hydrogliceric solution) and the preservative are added.

The above topical compositions, based on partly hydrolyzed fish gelatin according to the present invention, have been clinically tested in stomatology, ophthalmology, gynecology, dermatology and proctology, compared with analogous formulations without gelatin, in the following pathologies: dry mouth, dry eye, allergic conjunctivitis, atopic eczemas, vaginites, haemorrhoidal states.

The obtained results have shown that the treatments performed with the pharmaceutical forms above described are well tolerated and have particularly positive therapeutical perpectives, above all in stomatology and ophthalmology (as a salivary and lacrimal substitute) and in dermatology (in case of dystrophic ulcers and atopic dermatites).

Analogous comparative tests have been carried out between the above formulations and analogous formulations based on non-hydrolyzed or completely hydrolyzed gelatin.

The obtained results have surprisingly shown that the topical administration of the partly hydrolyzed fish gelatin according to the present invention provides a significantly higher therapeutic effect.

It is therefore a further object of the present invention a topical pharmaceutical composition, wherein said composition is characterized in that it includes, as active ingredient, a quantity of partly hydrolyzed fish gelatin as described hereinabove and in the appended claims.

Depending on the type of pathology to be topically treated, said composition can further include one or more active ingredients other than the partly hydrolyzed fish gelatin and, optionally, one or more additives, excipients, carriers, preservatives, as described hereinabove and in the appended claims. Considering the toxicological problems connected to the use of preservatives, the present invention also foresees the possibility of avoiding the use thereof by employing preparations to be prepared from time to time at the moment of use, in reduced quantities to be consumed within the following 12-24 hours. For this purpose the use of tablets or powders to be dissolved at the moment of use is provided.

In the light of what above described, it is a further object of the present invention a pharmaceutical composition including, as an active ingredient, the partly hydrolyzed fish gelatin according to what previously described and according to what reported in the appended claims, for use as a topical medicament.

## Claims

1. A pharmaceutical composition comprising type A fish gelatin for use in the topical treatment of oropharyngeal and gingival, ocular, gynaecological, dermatological, proctological, otologic and rhinological pathologies.

2. The composition for use according to claim 1, wherein said type A fish gelatin has an average molecular weight of 5,000 D.

3. The composition for use according to claim 1, wherein said type A fish gelatin is present in a quantity between 0.05% to 10% by weight based on the total weight of the composition, preferably, between 0.1% and 5% by weight.

4. The composition for use according to any one of the preceding claims, further including at least an active ingredient chosen among antibiotics, cortisone drugs, antihistamines, vasoconstrictors, vitamins, retinoides.

5. The composition for use according to any one of the preceding claims, further including at least a pharmaceutically acceptable addictive, excipient, carrier, preservative.

6. The composition for use according to any one of claims 1 to 5, for the topical treatment of patho-logical states of the oropharyngeal and ocular mucosae chosen among dry mouth, dry eye, allergic conjunctivites.

7. The composition for use according to claim 1 or 6, in the form of a solution to be used as a salivary or lacrimal substitute.

8. The composition for use according to claim 1 wherein said gynaecological pathologies are vaginites.

9. The composition for use according to claim 1 or 8 in form of douche or pessary.

10. The composition for use according to claim 1, wherein said dermatological pathologies are dystrophic ulcers, dermatites, atopic eczemas, sunburns.

11. The composition for use according to claim 1 or 10 in the form of cream.

12. The composition for use according to claim 11, wherein said cream further includes at least a vitamin.

13. The composition for use according to claim 12, wherein said vitamin is selected from the group including vitamin A, E, C.

14. The composition for use according to any one of the claims 11 to 13, wherein said cream further includes at least a cicatrizant substance.

15. The composition for use according to claim 1, wherein said proctological pathologies are haemorrhoid, rhagades, proctites.

## Patentansprüche

1. Pharmazeutische Zusammensetzung, umfassend eine Fischgelatine vom Typ A zur Verwendung bei der topischen Behandlung von Krankheiten im Mundrachenraum, Erkrankungen des Zahnfleischs, der Nase und Nasennebenhöhlen, der Augen, der Ohren, gynäkologischen, dermatologischen und proktologischen Erkrankungen.

2. Zusammensetzung zur Verwendung nach Anspruch 1, wobei die Fischgelatine vom Typ A ein Gewichtsmittel des Molekulargewichts von 5000 P aufweist.

3. Zusammensetzung zur Verwendung nach Anspruch 1, wobei die Fischgelatine vom Typ A in einer Menge zwischen 0,05 % und 10 % Gewichtsanteil vorhanden ist, basierend auf dem Gesamtgewicht der Zusammensetzung, vorzugsweise zwischen 0,1 % und 5 % Gewichtsanteil.

4. Zusammensetzung zur Verwendung nach einem der vorhergehenden Ansprüche, ferner umfassend mindestens eine Wirkstoffzutat, ausgewählt aus Antibiotika, Cortisonmedikamenten, Antihistaminika, Vasokonstriktoren, Vitaminen, Retinoiden.

5. Zusammensetzung zur Verwendung nach einem der vorhergehenden Ansprüche, ferner umfassend mindestens einen pharmazeutisch akzeptablen Zusatzstoff, Arzneistoffträger, Träger, Konservierungsstoff.

6. Zusammensetzung zur Verwendung nach einem der Ansprüche 1 bis 5 zur topischen Behandlung von Krankheiten der Mund-/Rachen- und Augenschleimhaut, ausgewählt aus trockenem Mund, trockenen Augen, allergischer Konjunktivitis.

7. Zusammensetzung zur Verwendung nach Anspruch 1 bis 6 in Form einer Lösung, um als Speichel- oder Tränenersatz verwendet zu werden.

8. Zusammensetzung zur Verwendung nach Anspruch 1, wobei es sich bei den gynäkologischen Erkrankungen um Scheidenentzündungen handelt.

9. Zusammensetzung zur Verwendung nach Anspruch 1 oder 8 als Intimdusche oder Vaginalzäpfchen.

10. Zusammensetzung zur Verwendung nach Anspruch 1, wobei es sich bei den dermatologischen Erkrankungen um dystrophe Ulzera, Dermatitis, atopische Ekzeme, Sonnenbrände handelt.

11. Zusammensetzung zur Verwendung nach Anspruch 1 oder 10 in Form einer Creme.

12. Zusammensetzung zur Verwendung nach Anspruch 11, wobei diese Creme zudem mindestens ein Vitamin umfasst.

13. Zusammensetzung zur Verwendung nach Anspruch 12, wobei dieses Vitamin ausgewählt ist aus der Gruppe, umfassend Vitamin A, E, C.

14. Zusammensetzung zur Verwendung nach einem der Ansprüche 11 bis 13, wobei diese Creme zudem mindestens ein Vernarbungsmittel umfasst.

15. Zusammensetzung zur Verwendung nach Anspruch 1, wobei es sich bei den proktologischen Erkrankungen um Hämorrhoiden, Rhagaden, Proktitis handelt.

## Revendications

1. Composition pharmaceutique comprenant de la gélatine de poisson de type A pour une utilisation dans le traitement topique de pathologies oro-pharyngées et gingivales, oculaires, gynécologiques, dermatologiques, proctologiques, otologiques et rhinologiques.

2. Composition pour une utilisation selon la revendication 1, dans laquelle ladite gélatine de poisson de type A possède un poids moléculaire moyen de 5 000 uma.

3. Composition pour une utilisation selon la revendication 1, dans laquelle la gélatine de poisson de type A est présente dans une quantité comprise entre 0,05 et 10 % en poids par rapport au poids total de la composition, de préférence entre 0,1 et 5 % en poids.

4. Composition pour une utilisation selon l'une quelconque des revendications précédentes, incluant de plus au moins un ingrédient actif choisi parmi des antibiotiques, des médicaments à base de cortisone, des antihistaminiques, des vasoconstricteurs, des vitamines et des rétinoïdes.

5. Composition pour une utilisation selon l'une quelconque des revendications précédentes, incluant de plus au moins un additif, un excipient, un vecteur de médicament ou un conservateur acceptable pharmaceutiquement.

6. Composition pour une utilisation selon l'une quelconque des revendications de 1 à 5, destinée au traitement topique d'états pathologiques des muqueuses oro-pharyngées et oculaires choisis parmi les suivants : xérostomie, kérato-conjonctivite sèche, conjonctivites allergiques.

7. Composition pour une utilisation selon les revendications 1 ou 6, sous forme d'une solution à utiliser comme un substitut salivaire ou lacrymal.

8. Composition pour une utilisation selon la revendication 1, dans laquelle lesdites pathologies gynécologiques sont des vaginites.

9. Composition pour une utilisation selon les revendications 1 ou 8 sous forme de poire à injection ou d'ovule.

10. Composition pour une utilisation selon la revendication 1, dans laquelle lesdites pathologies dermatologiques sont des ulcères dystrophiques, des dermatites, des eczémas atopiques et des érythèmes solaires.

11. Composition pour une utilisation selon les revendications 1 ou 10 sous forme de crème.

12. Composition pour une utilisation selon la revendication 11, dans laquelle ladite crème inclut de plus au moins une vitamine.

13. Composition pour une utilisation selon la revendication 12, dans laquelle ladite vitamine est sélectionnée dans le groupe comprenant les vitamines A, E et C.

14. Composition pour une utilisation selon l'une quelconque des revendications de 11 à 13, dans laquelle ladite crème inclut de plus au moins une substance cicatrisante.

15. Composition pour une utilisation selon la revendication 1, dans laquelle lesdites pathologies proctologiques sont des hémorroïdes, des rhagades et des proctites.
